# EUROPEAN PATENT SPECIFICATION

(11) **EP 3 407 773 B1**
(45) Date of publication and mention of the grant of the patent: **21.10.2020**
(21) Application number: 17704614.1
(22) Date of filing: 26.01.2017
(51) Int. Cl.: A61B 1/005, A61B 1/00, A61B 1/008

(54) **ENDOSCOPE DEVICE**
ENDOSKOPVORRICHTUNG
DISPOSITIF ENDOSCOPIQUE

(30) Priority: 27.01.2016 US 201662287667 P
(43) Date of publication of application: 05.12.2018
(73) Proprietor: Boston Scientific Scimed, Inc., Maple Grove, MN 55311 (US)
(72) Inventor: WEITZNER, Barry, Acton MA 01720 (US); DEVRIES, Robert, Northborough MA 02139 (US); ANDERSSON, Niklas, Wayland MA 01778 (US)
(74) Representative: Vossius & Partner Patentanwälte Rechtsanwälte mbB
(86) International application number: PCT/US2017/015146
(87) International publication number: WO 2017/132386

(56) References cited:
- AU-A1- 2013 251 209
- JP-A- 2011 110 244
- US-A1- 2003 069 475
- US-A1- 2004 267 093
- US-B2- 8 409 245

## Description

### Technical Field

Aspects of the present disclosure generally relate to medical devices. In particular, some aspects relate to an endoscopic device.

### Background

Endoscopes are used in a wide variety of diagnostic procedures to provide a physician with a view inside of a body. Many endoscopes are elongated, flexible elements with a distal portion that can be steered by a set of internal tension wires. Various control mechanisms are used to manipulate the tension wires. Some endoscopes have mechanical levers that can be operated by the physician to manipulate the wires, while other endoscopes have rotating knobs that manipulate the wires, permitting the portion of the scope to move in an upper-lower direction or a left-right direction.

These control mechanisms may be limited to movements in the upper-lower and left-right directions. Other control mechanisms are therefore required to control other portions of the endoscope and/or other tools, such as a probe attached to the distal portion of the endoscope. Other techniques are required to move the distal portion in other directions, such as any rotational movements that permit proper placement of a tool mounted on the distal portion, or translational movements that permit proper positioning of a tool extending out of a lumen of the endoscope. These numerous control mechanisms and techniques make many known endoscopes inefficient to operate by oftentimes requiring additional hands and/or operators.

JP 2011 110244 A discloses an electronic endoscope. The electronic endoscope includes a curving mechanism for curving an insertion tube distal end by a wire operation. The curving mechanism includes angle knobs rotatable around the rotation center axis; a plurality of pulleys to be interlocked with the insertion tube distal end via wires; a rotation transmitting means to be selectively engaged with one of the pulleys so as to transmit the rotation of the angle knob to the selected pulley; and a switch means for allowing the rotation transmitting means and one of the pulleys to move with respect to the other, so as to selectively switch the engagement between one of the pulleys and the rotation transmitting means.

US 2004/0267093 A1 discloses an electric bending endoscope that includes a bending portion, a bending driving mechanism which electrically bends the bending portion, an operation input member which instructs an operation to the bending driving mechanism so that the bending driving mechanism bends the bending portion, and a bending angle holding mechanism which holds the bending angle of the bending portion.

US 2003/0069475 A1 discloses an endoscope apparatus.

### SUMMARY

Aspects of the present disclosure relate to an endoscopic device. Numerous aspects of the present disclosure are now described. According to the invention, an endoscopic device as recited in the independent claim is provided. The dependent claims define embodiments.

One aspect is an endoscopic device. The device may comprise a shaft extending between a distal end and a proximal end. The shaft may include a controllable bend, a lumen, and a controllable channel movably set in the lumen. The device may further comprise a handle at the proximal end of the shaft. The handle may include a controller selectively engageable with the controllable bend and the controllable channel.

Aspects of the device may additionally and/or alternatively include any one or more of the following features. The controller may be selectively engageable with the controllable bend when not engaged with the controllable channel, and may be selectively engageable with the controllable channel when not engaged with the controllable bend. The controller may be a single, discrete controller for operation by a single hand. The controller may control movement of the controllable bend in an upper-lower direction, a left-right direction, an angular direction, or a combination thereof. The controller may control rotation of the controllable channel relative to the lumen. The controller may control translation of the controllable channel in a proximal-distal direction relative to the lumen. The device may further comprise an end effector adjacent a distal end of the controllable channel, wherein the controller may be selectively engageable with the end effector to activate the end effector. The end effector may be removably attached to the distal end of the controllable channel. The controller may control movement of the end effector relative to the controllable channel.

The device may further comprise a tool in the controllable channel. The tool may include an end effector at a distal end of the tool, wherein the tool may be movable within the controllable channel in a distal direction to extend the end effector out of the controllable channel, and a proximal direction to retract the tool into the working channel. The controller may be selectively engageable with the tool to control movement of the tool and activate the end effector. The device may further comprise an imaging device on a distal face of the shaft, wherein the controller is selectively engageable with the imaging device to control activation of the imaging device. The controller may be located on an upper surface of the handle and be operable with a thumb. The device further comprises a selector for selectively engaging the controller with the controllable bend or the controllable channel, wherein the selector is located on a side surface or a lower surface of the handle and operable with one or more fingers. The selector may comprise a plurality of buttons and the controller may be selectively engageable by activating one of the plurality of buttons.

There is also disclosed a handle for an endoscopic device with a shaft including a plurality of operative elements. The handle may comprise a handle body and a controller movably mounted on the handle body, the controller being operable with a plurality of sensors to generate a directional signal when the controller is moved. The handle may further comprise a selector on the handle body, the selector being operable to engage the controller with one of the plurality of operative elements and generate a switching signal. A processor within the handle body may be configured to activate one or more of the plurality of operative elements in response to the directional signal and the switching signal.

The handle may additionally and/or alternatively include any one or more of the following features. The handle may be removably attachable to the shaft. The handle may further comprise one or more power sources for the plurality of sensors, the processor, and the plurality of operative elements. The controller and the selector may be arranged on the handle body for single-handed operation.

There is also described a method for operating an endoscope device with a handle including a selector and a controller, which does not form part of the invention. The method may comprise the steps of using the selector to engage the controller with a first operative element of the device; and operating the first operative element with the controller. Other steps may comprise using the selector to engage the controller with a second operative element of the device; and operating the second operative element with the controller.

The method may additionally and/or alternatively include any one or more of the following features. The controller may be engaged with the second operative element when the controller is not engaged with the first operative element, and the controller may be engaged with the first operative element when the controller is not engaged with the second operative element. The method may further comprise performing the method with a single hand. The first operative element may include an actuator configured to bend a controllable bend of a shaft of the endoscopic device, wherein the first operating step may comprise moving the controller in a direction to bend the shaft in a corresponding direction. The second operative element may include an actuator configured to move a controllable channel movably set in a lumen of the shaft, wherein the second operating step may comprise moving the controller in a proximal-distal direction to translate the controllable channel in a corresponding direction relative to the lumen.

It may be understood that both the foregoing summary and the following detailed descriptions are exemplary and explanatory only, neither being restrictive of the inventions claimed below.

### BRIEF DESCRIPTION OF THE DRAWINGS

The accompanying drawings, which are incorporated in and constitute a part of this specification, illustrate exemplary aspects that, together with the written descriptions, serve to explain the principles of this disclosure.
FIG. 1 depicts an exemplary endoscopic device according to the present disclosure, the device including a handle and a shaft.
FIG. 2 depicts a view of the handle of FIG. 1 when grasped by a hand.
FIG. 3 depicts a section view of the handle and shaft of FIG. 1.
FIG. 4 depicts a set of exemplary controllable bends on the shaft of FIG. 1.
FIG. 5 depicts a view of a distal face of a shaft of FIG. 1.
FIG. 6 depicts a section view of an exemplary controllable channel within a lumen of the shaft of FIG. 1.
FIG. 7A depicts a section view of the distal end of the shaft of FIG. 1.
FIG. 7B depicts another section view of the distal end of the shaft of FIG. 1.
FIG. 8 depicts a section view of an exemplary imaging device on the distal end of the shaft of FIG. 1.
FIG. 9 depicts an exemplary method for operating the endoscopic device of FIG. 1 according to the present disclosure.

### DETAILED DESCRIPTION

The present disclosure is now described with reference to exemplary aspects of an endoscopic device and method. Some aspects are depicted and/or described with reference to an endoscope having specific features, such as a shaft with a controllable bend, or a controllable channel movably set in a lumen of the shaft. These references are provided for convenience and not intended to limit the present disclosure unless incorporated into the appended claims. Accordingly, the concepts and novelty underlying each aspect may be utilized for any analogous type of device or method, medical or otherwise.

A number of opposing directional terms are used in the present disclosure, such as "proximal" ("P") opposite of "distal" ("D"), left ("L") opposite of right ("R"), and "upper" ("U") opposite of "lower" ("Lo"). As used herein, the term proximal refers to a position closer to a hand of a user, whereas the term distal refers to a position further from the hand. In some instances, the term upper may refer to a position closer to a thumb of the hand, whereas the term lower may refer to a position further from the thumb. These directional terms are provided for convenience. Unless claimed, they are not intended to limit the present disclosure to a particular direction or orientation.

As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such process, method, article, or apparatus. Unless stated otherwise, the term "exemplary" is used in the sense of "example," rather than "ideal."

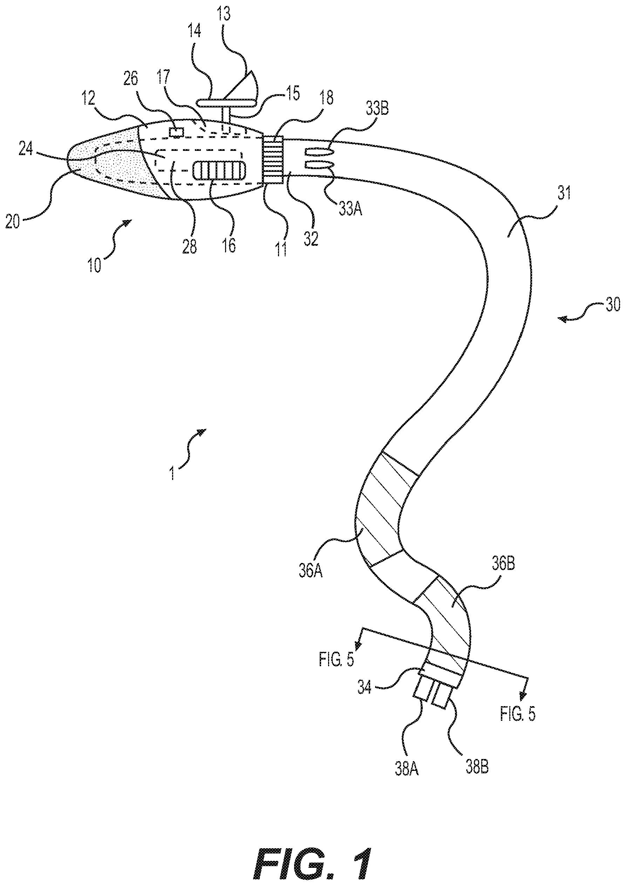

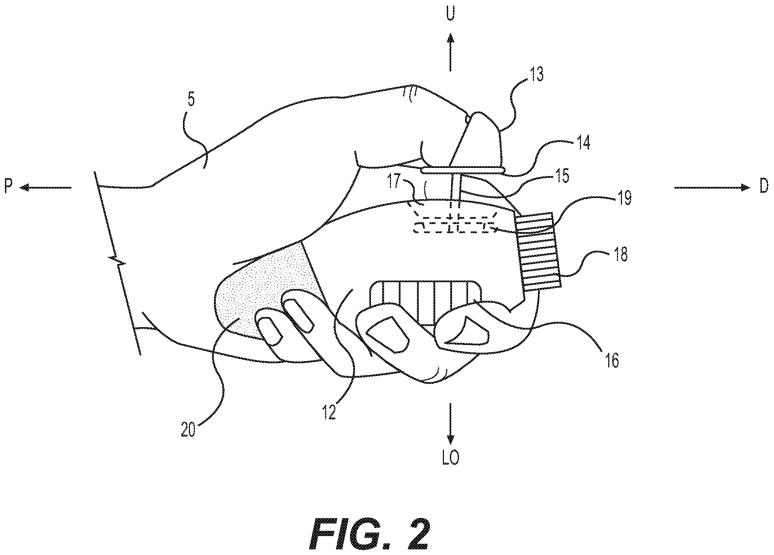

One aspect of the present disclosure is depicted in FIG. 1 as an endoscopic device 1 including a handle 10 and a shaft 30. A distal end 11 of handle 10 has a shaft interface 18 attached to a proximal end 32 of shaft 30. Handle 10 includes a controller 14 and a selector 16 that, in one aspect, is arranged for one-handed operation so as to provide the physician with a free hand. As depicted in FIG 2, handle 10 has an upper surface opposite of a lower surface. Controller 14 is located on the upper surface and may be operable with a thumb, while selector 16 is located on a side surface and may be operable with an index finger and/or a middle finger. An optional thumb strap 13 is provided to secure the thumb to controller 14. When grasped by a hand 5, a gripping surface 20 of handle 10 may be pushed into the palm and secured therein by a ring finger and/or a pinky finger.

Controller 14 is a single, discrete controller that may be operable with a single hand. As shown in FIGs. 1-3, controller 14 includes a joystick 15 that extends outwardly from an interior space 24 of handle 10 through an opening in an upper surface of handle 10. Joystick 15 is pivotally mounted in a recess 17 that has a plurality of sensors 19 mounted therein. The plurality of sensors 19 are depicted as an annular array of motion sensors mounted inside of interior portion 24 and operable with a lower end of joystick 15 to generate a directional signal ("DS") when controller is moved in any direction relative to recess 17. Joystick 15 may be moved in any direction parallel to a plane defined by recess 17, such as a proximal or distal direction (a "proximal-distal direction"), a left or right direction (a "left-right direction"), an angular direction, or a combination thereof. Joystick 15 may also be moved in any direction perpendicular to the plane defined by recess 17, including an upper or lower direction (an "upper-lower direction"), as well as an upper-lower direction combined with any of the above directions. Sensors 19 are configured to generate a directional signal from any combination of these movements.

Selector 16 is used to selectively engage controller 14 with one of a plurality of operative elements of device 1. For example, controller 14 may be selectively engaged with a first operative element when not engaged with a second operative element, and may be selectively engaged with the second operative element when not engaged with the first operative element. As shown in FIGs. 1 and 2, selector 16 is a switching element having a plurality of buttons. A total of seven buttons are shown. Each button is associated with one of the plurality of operative elements, such that depressing a button generates a switching signal ("SS") specific to the operative element associated therewith. One button may be used to associate the directional signals generated by sensors 19 with (or "select") an operative element that bends a portion of shaft 30, while another button selects an operative element that extends a controllable channel out of a lumen in shaft 30, and yet another button selects an operative element that extends a tool out of the controllable channel. More specific uses for each button are described in detail below.

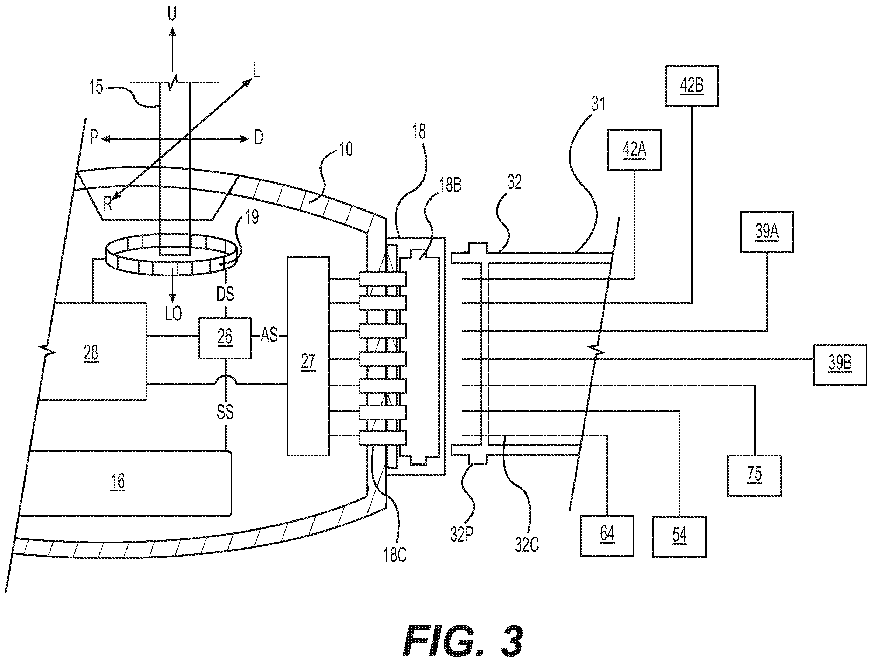

Each of selector 16 and sensors 19 are coupled to a processor 26 housed in handle 10. Processor 26 of FIG. 3, for example, is configured to receive the directional signals from sensors 19 and the switching signals from selector 16, generate an activation signal ("AS") therefrom, and transmit the activation signal to a switching element 27 housed in handle 10. Switching element 27 is coupled to processor 26 and a power source 28, which may be a battery housed in handle 10. In operation, switching element 27 receives the activation signal from processor 26 and powers the operative element selected by selector 16. By virtue of this configuration, controller 14 may be used to activate or control any operative element of device 1. The aforementioned coupling may be achieved through any wired or wireless means, including the examples described herein.

As shown in FIG. 1, shaft 30 has a flexible shaft body 31 extending between a proximal end 32 and a distal end 34. Proximal end 32 may be removably attached to a shaft interface 18 of handle 12 by a bayonet mount, wherein an annular surface of proximal end 32 has one or more protrusions 32P extending outwardly therefrom Each protrusion 32P is engageable with a corresponding annular groove 18G on shaft interface 18. A biasing element may be used to secure each protrusion 32P in a groove 18G. With shaft 30 attached thereto, handle 10 may be used to guide or snake shaft body 31 through a body. Shaft interface 18 of FIG. 3 has a set of electrical contacts 18C coupled with power source 28 by switching element 27. Each contact 18C is conductively engageable with a corresponding electrical contact 32C on proximal end 32, such that a plurality of wires may be routed inside shaft body 31, from contacts 32C, to power any operative element of shaft 30.

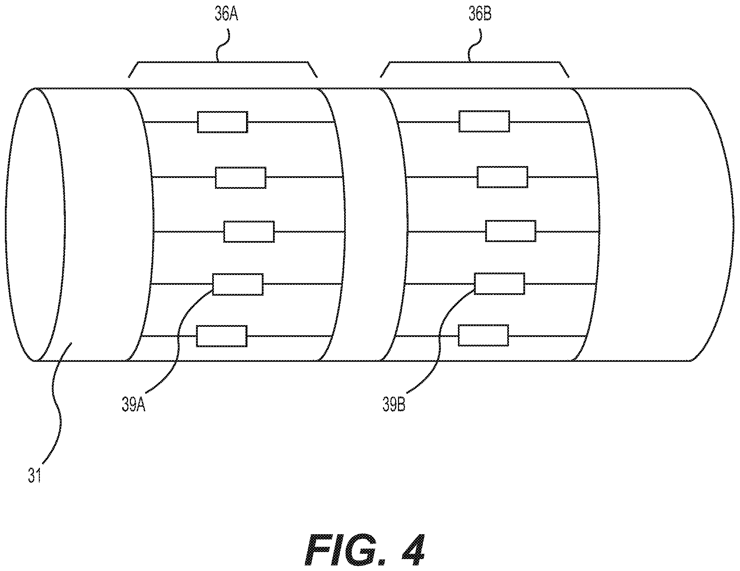

The operative elements of shaft 30 include one or more controllable bends. As shown in FIGs. 1 and 4, shaft body 31 has two controllable bends 36A and 36B, each having one or more actuators 39A and 39B positioned within or around an exterior perimeter of shaft body 31. Each actuator 39A and 39B may, for example, be an electric actuator that may be wired to one or more of contacts 32C, such as a piezoelectric actuator, a shape memory actuator, and/or an electroactive polymer actuator. Exemplary actuators 39A and 39B are described in U.S. Patent No. 8,517,924. As described therein, and illustrated in FIG. 4, each actuator 39A, 39B may be a conductive element that contracts or expands in response to an amount of electricity from power source 28, thereby applying a tensile and/or compressive force to shaft body 31 that moves controllable bend 36A or 36B in a particular direction. For example, actuators 39A and 39B may be used to bend distal end 34 of shaft 30 in an upper-lower direction, a left-right direction, an angular direction, or a combination thereof.

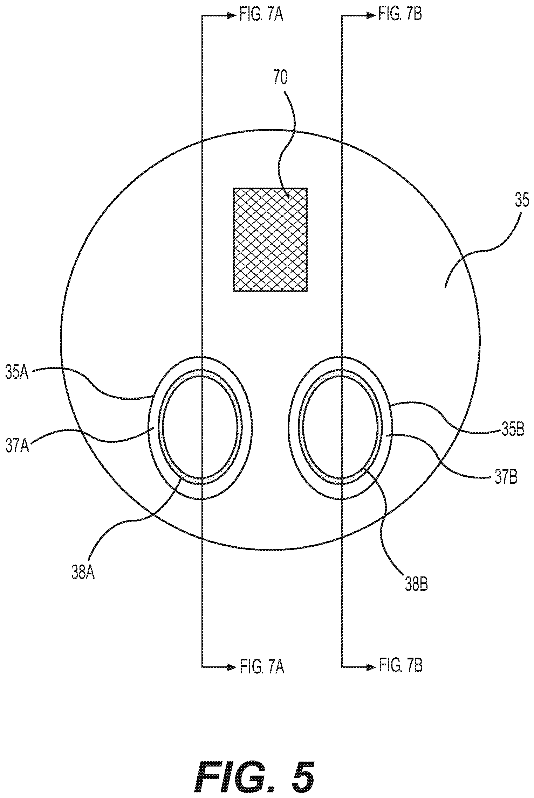

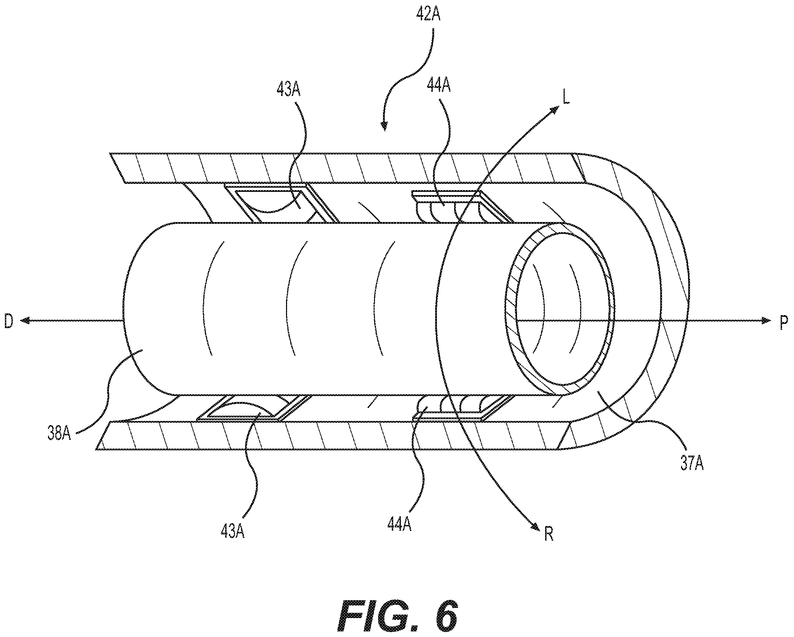

Shaft 30 may have one or more lumens extending therethrough. As shown in FIGs. 1 and 5, for example, shaft 30 has a first lumen 37A and a second lumen 37B. Each lumen 37A, 37B extends between a proximal opening 33A, 33B adjacent proximal end 32 (FIG. 1), and a distal opening 35A, 35B on a distal face 35 of shaft body 31 (FIG. 5). Each lumen 37A and 37B may house another operative element of device 1. For example, each of the first and second lumens 37A and 37B has, respectively, a first controllable channel 38A and a second controllable channel 38B movably set therein. Channels 38A and 38B are moveable relative to lumens 37A and 37B. According to one aspect, a first set of actuators 42A (FIGs. 3 and 6) and a second set of actuators 42B (FIG. 3) are provided to move channels 38A and 38B within lumens 37 and 37B. Actuators 42A and 42B may be wired to one of contacts 32C and housed in an interior portion of shaft body 31. As shown in FIG. 6, the first set of actuators 42A include an opposing pair of motorized rollers 43A mounted in lumen 37A for translational movement of channel 38A in the proximal or distal direction along a longitudinal axis of lumen 37A, and an opposing pair of motorized rollers 44A mounted in lumen 37A for rotational movement of channel 38A in a left (clockwise) or right (counterclockwise) direction about the longitudinal axis of lumen 37A. Although not shown in FIG. 6, the second set of actuators 42B may be identical to the first set of actuators 42A. In this way, each controllable channel 38A, 38B may be rotated and/or translated independently within lumen 37A or 37B, as well as moved with distal end 34 of shaft 30 in an upper-lower direction, a left-right direction, an angular direction, or a combination thereof.

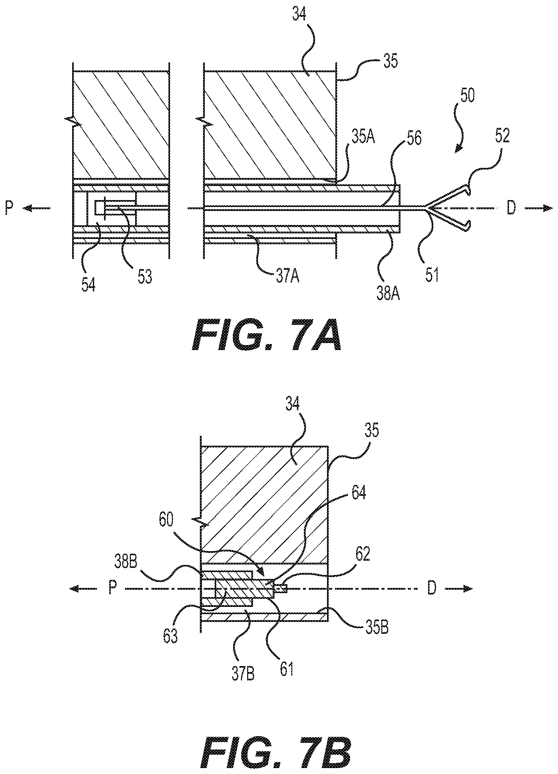

Other operative elements of device 1 may be housed in controllable channels 38A and 38B. Two examples are illustrated in FIGs. 7A-B, wherein a first tool 50 is housed within channel 38A (FIG. 7A), and a second tool 60 is housed within channel 38B (FIG. 7B). Controller 14 may be selectively engaged with either of tool 50 or tool 60, as described further below.

First tool 50 of FIG. 7A has a distal end 51 with an end effector 52, a proximal end 53 with an actuator 54, and an elongated rod 56 extending between distal and proximal ends 51, 53. End effector 52 is depicted as having, for example, a set of jaws biased toward an open position. In one aspect, the distal end 51 of tool 50 is inserted through proximal opening 33A of shaft 30 (FIG. 1) until the proximal end 53 of tool 50 is positioned in channel 38A. Rod 52 is moveable in the proximal-distal direction relative to proximal end 53 by actuator 54, which may be powered by an electrical contact wired to one of contacts 32C (FIG. 3). Controller 14 may be used to activate an element of tool 50, such as end effector 52, when selectively engaged with actuator 54 by selector 16. For example, once engaged by selector 16, controller 14 may be moved distally, causing actuator 54 to move rod 56 distally and expand the jaws of end effector 52 out of channel 38A; or proximally, causing actuator 54 to move rod 56 proximally and collapse said jaws into channel 38A.

Second tool 60 of FIG. 7B has a distal end 61 with an end effector 62 and a proximal end 63 with an actuator 64. End effector 62 is depicted as having, for example, a blade. As shown in FIG. 7B, proximal end 63 of tool 60 may be inserted into the distal end of controllable channel 38B and secured therein. For example, an annular surface of proximal end 63 may have a protrusion that is snapped into a corresponding groove on the interior surface of channel 38B. The blade may, for example, be rotated at high-speed by actuator 64, which may be powered by an electrical contact in channel 38B that is wired to one of contacts 32C, or rotated at a low speed by rotating controllable channel 38B in lumen 37B. Thus, as before, controller 14 may be used to activate end effector 62 when engaged with actuator 64 by selector 16. For example, controller 14 may be moved proximally, causing actuator 64 to rotate the blade; or distally, causing actuator 64 to stop or slow the blade.

Tools 50 and 60 may be rotated and/or translated independently within each controllable channel 38A and 38B, as well as moved with distal end 34 of shaft 30 in an upper-lower direction, a left-right direction, and any combination thereof. The degrees of freedom provided by this configuration permit each end effector 52 and 62 to be located at a targeted location within a body. In some aspects, as with blade 62, the translational and/or rotational movements provided by controllable channels 38A and 38B may enable the operation of tool 50 or 60.

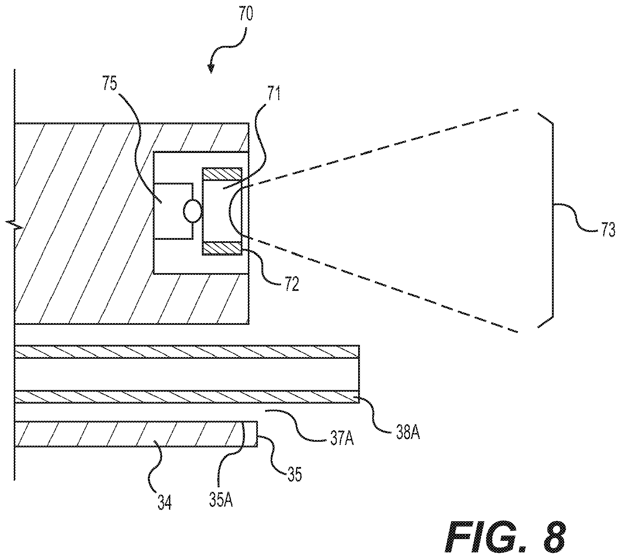

Another operative element of shaft 30 may comprise an imaging device 70 mounted on distal face 35 (FIGs. 5 and 8). Imaging device 70 has a camera 71 and a light source 72. As shown in FIG. 8, controllable channel 38A, for example, may be moved distally out of lumen 37A and into a field of view 73 of camera 71. An actuator 75 is provided to adjust field of view 73 by moving camera 71 and light source 72 relative to distal face 35. Device 70 and actuator 75 may be wired to one of contacts 32C so that controller 14 may be used to operate imaging device 70 when engaged with actuator 75 by selector 16. For example, controller 14 may be moved in a proximal-distal direction and/or a left-right direction to adjust field of view 73 by pivoting camera 71 and light source 72 in a corresponding upper-lower direction and/or a left-right direction. As a further example, controller 14 may also be moved like a push-button in the upper direction to activate or deactivate camera 71, or the lower direction to activate or deactivate light source 72. Similar movements may be used to adjust the focus of camera 71, or to adjust the amount of light emitted by light source 72.

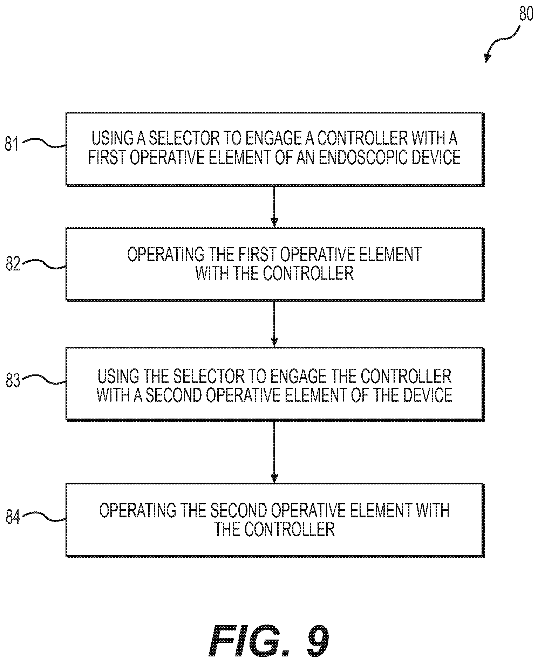

Numerous methods, which do not form part of the invention are now described with reference to FIG. 9. An exemplary method 80 for operating endoscope device 1 is disclosed. Method 80 comprises a step 81 of using selector 16 to engage controller 14 with a first operative element of shaft 30. In the illustrated aspect, step 81 is performed by pushing a first one of the plurality of buttons of selector 16 shown in FIG. 1. Step 81 may be used to selectively engage controller 14 with any operative element described herein, such as controllable bends 36A and 36B, controllable channels 38A and 38B, tools 50 and 60, imaging device 70, and/or any actuator used to operate these elements. Another step 82 comprises operating the first operative element with controller 14. For example, controllable bend 36A may be operated by moving controller 14 in a direction to bend shaft 30 in a corresponding direction. Processor 26 may be used to dictate any correspondence between the directional signal generated by sensors 19 and the actual movement of any operative element, such as controllable bend 36A. The correspondence may, for example, be synchronized, such that a left-right directional movement of controller 14 corresponds to a left-right directional movement/bending of bend 36A.

Another step 83 of method 80 comprises using selector 16 to engage controller 14 with a second operative element of shaft 30. Step 83 may be used to selectively engage controller 14 with any operative element described herein. Step 83 may be performed by pushing a second one of the plurality of buttons shown in FIG. 1. Another step 84 comprises operating the second operative element with controller 14. For example, once bend 36A has been moved in the desired direction, then controllable channel 38B may be translated by engaging controller 14 with the second set of actuators 42B, and moving controller 14 in a direction to translate channel 38B in a corresponding direction. The correspondence between a directional signal for one operative element need not align with correspondence of another. For example, moving controller 14 in the left direction within operative step 82 may move bend 36A in the left direction, while moving controller 14 in a proximal direction with operative step 84 may rotate controllable channel in a clockwise direction.

With slight modification, method 80 may also be used to operate tool 50, tool 60, and imaging device 70. For example, other method steps may comprise a step for using selector 16 to engage controller 14 with actuator 54, 64, or 75; and a step for operating actuators 54, 64, or 75 with controller 14 to accomplish any movements of tool 50, tool 60, or imaging device 70 described above. Additional method steps may comprise positioning end effectors 52 or 62 at a target point in a body, and selectively engaging controller 14 with actuators 54 or 64 to activate end effectors 52 or 62. Given various arrangements of controller 14 and selector 16 described above, it should be appreciated that any aspect of method 80 may be performed with a single hand.

Numerous alternative aspects of device 1 and method 80 are now described with reference to FIGs. 1-8. Each alternative aspect may include features that modify or enhance a feature of device 1 or a step of method 80. Any feature of any alternative aspect may be combined with any feature of device 1 and method 80 described herein, each possible variation being part of the present disclosure.

Handle 10 is described as being configured for single-handed operation, but this is not required. For example, the size of handle 10 and the arrangement of controller 14 and selector 16 may be modified to produce a handle 10 configured for two-handed or two-operator operation. Gripping surface 20, for example, may be grasped by one hand while controller 14 and selector 16 are manipulated by the other hand. An exemplary arrangement of controller 14 and selector 16 within hand 5 is depicted in FIG. 2; however, hand 5 may grasp handle 10 in any way that permits operation of controller 14 and selector 16 with any digit of any hand.

Controller is described with a joystick, but this is not required. For example, controller 14 may alternatively be a directional pad, a touch pad, a plurality of buttons, or like means. Plurality of sensors 19 are described as motion sensors for generating a directional signal by tracking a lower end of joystick 15. Any equivalent sensing technology may be used to generate the directional signal. Joystick 19 may also be omitted in some aspects. For example, joystick 19 may be replaced by a static track pad that generates a direction signal when a thumb is slid thereon. As a further example, sensors 19 may also be configured to track the location of handle 10 in a space, such that an equivalent directional signal may be generated by moving the handle 10 in a particular direction relative to a user, without joystick 15, or in combination therewith.

Selector 16 is described as a plurality of buttons. Seven buttons are depicted in FIG. 1, for example, one for each of actuators 39A, actuators 39B, actuators 42A, actuators 42B, actuator 54, actuator 64, and actuator 75. Any number of buttons may be provided. Likewise, any of these buttons may be consolidated or arranged in any manner. Selector 16 need not have any buttons at all and may, instead, be a selection pad, a selection wheel, a microphone for voice control, a transceiver for computer control, or like means. The location of selector 16 may also vary. For example, selector 16 may alternatively be located on any surface of handle 10, or on shaft 30 adjacent its proximal end 32.

Handle 10 is described as removably attached to shaft 30 by a shaft interface 18. Handle 10 may, thus, be a reusable device while shaft 30 is a limited or even single use device. The described bayonet mount for connecting handle 10 to shaft 30 is merely exemplary as any known type of attachment may be used, moveable or permanent. Said attachment may also be used to maintain a sterile barrier between handle 10 and shaft 30, such as a polymeric barrier surrounding handle 10, thereby protecting handle 10 from contamination. Of course, handle 10 may also be integral with shaft 30 in a monobody configuration of device 1, thereby omitting shaft interface 18 entirely; in which case, electrical contacts 18C and 32C may be wired within device 1.

One or more power sources 28 is described above as being located in handle 10 and wired to various operative elements of device 1. Any number of power sources may be provided. For example, a first power source may provided in handle 10 to power each element housed therein, while a second power source is provided in shaft 30 to power each element housed therein. Alternatively, certain elements of device 1, such as tools 50 and 60 for example, may comprise their own operative power sources to promote interchangeability of those elements.

Shaft 30 is described as having two controllable bends 36A and 36B, each being operated by one or more electric actuators 39A or 39B (FIG. 4). Any number of controllable bends may be provided on shaft 30, each of which may be operated by any type of actuator, electric or otherwise. For example, a set of Bowden wires, or other mechanical force transfer means, may be provided on or within shaft 30 and operated by one or more electrical motors to move any portion of shaft 30 as described herein. Shaft 30 is also described as having two lumens 37A and 37B, yet any number of lumens may be provided. A controllable channel 38A or 38B is provided in each lumen 37A, 37B, although this is not required. For example, either lumen 37A may remain open for use as a fluid delivery means, wherein a fluid source is attached to opening 33A, or as a suction means. Either of channels 38A and 38B may be configured for a similar purpose, thereby allowing fluid or suction to be provided at a targeted location in a body when channel 38A or 38B is moved.

First set of actuators 42A is described, for example, as an opposing pair of motorized rollers 43A and 44A configured to translate and/or rotate controllable channels 38A. Any type of actuator may be used to translate or rotate either of channels 38A and 38B. Although described as sets or pairs, a single actuator may also be configured to translate and/or rotate channels 38A, 38B. For example, a single actuator may be coupled one or more gears that convert a single force, such as a rotational force, into one or more forces for translating and/or rotating channels 38A and 38B. Actuator 54 for tool 50, actuator 64 for tool 60, and actuator 75 for imaging device 70 may also be any type of actuator. Aspects of end effectors 52 and 62 are also exemplary, such that tools 50 and 60 may include any type of end effector, any of which may be activated by controller 14. In some aspects, either of tools 50 or 60 may be manually operated. For example, a proximal portion of rod 56 of tool 50 may extend out of proximal opening 33A for manual operation.

Various operative elements of shaft 30 are also described as being wired to an element of handle 10, such as controller 14 and selector 16. It is contemplated that any of these connections may also be wireless. For example, each actuator 39A-B, 42A-B, 54, 64, and/or 75 may have a wireless transceiver in communication with a wireless transceiver of handle 10 that is coupled to controller 14 and/or selector 16, thereby allowing any element of device 1 to be controlled wireless. In this regard, the connection between handle 10 may be used exclusively to provide power and a stable operating platform for shaft 30. Alternatively, if each of these actuators comprises its own motor and/or power sources, then the connection between handle 10 and shaft 30 may be configured such that at least handle 10 is fully encased in a sterile field to prevent contamination and promote re-use.

While principles of the present disclosure are described herein with reference to illustrative aspects for particular applications, it should be understood that the disclosure is not limited thereto. Those having ordinary skill in the art and access to the teachings provided herein will recognize additional modifications, applications, aspects, and substitution of equivalents all fall within the scope of the aspects described herein. Accordingly, the present disclosure is not to be considered as limited by the foregoing description.

## Claims

1. An endoscopic device, the device comprising:
a shaft (30) extending between a distal end and a proximal end (32), the shaft (30) including a controllable bend (36A, 36B), a lumen (37A, 37B), and a controllable channel (38A, 38B) movably set in the lumen (37A, 37B);
a handle (10) at the proximal end (32) of the shaft (30), the handle (10) including a controller (14) selectively engageable with the controllable bend (36A, 36B) and the controllable channel (38A, 38B); and
a selector (16) for selectively engaging the controller (14) with the controllable bend (36A, 36B) or the controllable channel (38A, 38B).

2. The device of claim 1, wherein the controller (14) is selectively engageable with the controllable bend (36A, 36B) when not engaged with the controllable channel (38A, 38B) and selectively engageable with the controllable channel (38A, 38B) when not engaged with the controllable bend (36A, 36B).

3. The device of any preceding claim, wherein the controller (14) is a single, discrete controller operable with a single hand.

4. The device of any preceding claim, wherein the controller (14) controls movement of the controllable bend (36A, 36B) in an upper-lower direction, a left-right direction, an angular direction, or a combination thereof relative to the handle (10).

5. The device of any preceding claim, wherein the controller (14) controls rotation of the controllable channel (38A, 38B) relative to the lumen (37A, 37B).

6. The device of any preceding claim, wherein the controller (14) controls the controllable channel (38A, 38B) to move in a proximal-distal direction relative to the lumen (37A, 37B).

7. The device of any preceding claim, further comprising an end effector (52, 62) adjacent a distal end of the controllable channel (38A, 38B), wherein the controller (14) is selectively engageable with the end effector (52, 62) to activate the end effector (52, 62).

8. The device of claim 7, wherein the controller (14) controls movement of the end effector (52, 62) relative to the controllable channel (38A, 38B).

9. The device any one of claims 1 to 6, further comprising:
a tool (50, 60) in the controllable channel (38A, 38B), the tool including an end effector (52, 62) at a distal end (51, 61) of the tool,
wherein the tool (50, 60) is movable within the controllable channel (38A, 38B) in a distal direction to extend the end effector (52, 62) out of the controllable channel (38A, 38B) and a proximal direction to retract the tool (50, 60) into the controllable channel (38A, 38B), and
wherein the controller (14) is selectively engageable with the tool (50, 60) to control movement of the tool (50, 60) and activate the end effector (52, 62).

10. The device of any preceding claim, wherein the selector (16) comprises a plurality of buttons, and the controller (14) is selectively engaged by activating one of the plurality of buttons.

11. The device of any preceding claim,
wherein the controller (14) is movably mounted on an upper surface of the handle (10) and operable with a plurality of sensors (19) to generate a directional signal when the controller (14) is moved,
wherein the selector (16) is configured to generate a switching signal to selectively engage the controller (14) with the controllable bend (36A, 36B) or the controllable channel (38A, 38B), and
wherein the device further comprises a processor (26) configured to activate the controllable bend (36A, 36B) or the controllable channel (38A, 38B) in response to the directional signal and the switching signal.

12. The device of claim 11, further comprising a power source (28) for the plurality of sensors (19), the processor (26), the controllable bend (36A, 36B), and the controllable channel (38A, 38B).

13. The device of any preceding claim, wherein the controllable bend (36A, 36B) has a first actuator (39A, 39B) and the controllable channel (38A, 38B) has a second actuator (42A, 42B), and wherein the device is configured to selectively activate the first actuator (39A, 39B) or the second actuator (42A, 42B) in dependence on a switching signal from the selector (16).

14. The device of any preceding claim, further comprising an imaging device (70) on a distal face of the shaft (30), wherein the controller (14) is selectively engageable with the imaging device (70) to activate the imaging device (70).

15. The device of any preceding claim, wherein the handle (10) is configured for single-handed operation.

## Patentansprüche

1. Endoskopvorrichtung, wobei die Vorrichtung aufweist:
einen Schaft (30), der sich zwischen einem distalen Ende und einem proximalen Ende (32) erstreckt, wobei der Schaft (30) eine steuerbare Biegung (36A, 36B), ein Lumen (37A, 37B) und einen steuerbaren Kanal (38A, 38B) aufweist, der im Lumen (37A, 37B) beweglich eingesetzt ist;
einen Griff (10) am proximalen Ende (32) des Schafts (30), wobei der Griff (10) ein Steuerteil (14) aufweist, das mit der steuerbaren Biegung (36A, 36B) und dem steuerbaren Kanal (38A, 38B) selektiv in Eingriff bringbar ist; und
einen Selektor (16) zum selektiven In-Eingriffbringen des Steuerteils (14) mit der steuerbaren Biegung (36A, 36B) oder dem steuerbaren Kanal (38A, 38B).

2. Vorrichtung nach Anspruch 1, wobei das Steuerteil (14) mit der steuerbaren Biegung (36A, 36B) selektiv in Eingriff bringbar ist, wenn es nicht im Eingriff mit dem steuerbaren Kanal (38A, 38B) steht, und mit dem steuerbaren Kanal (38A, 38B) selektiv in Eingriff bringbar ist, wenn es nicht im Eingriff mit der steuerbaren Biegung (36A, 36B) steht.

3. Vorrichtung nach einem der vorstehenden Ansprüche, wobei das Steuerteil (14) ein einzelnes, diskretes Steuerteil ist, das mit einer Hand bedienbar ist.

4. Vorrichtung nach einem der vorstehenden Ansprüche, wobei das Steuerteil (14) Bewegung der steuerbaren Biegung (36A, 36B) in Aufwärts-Abwärts-Richtung, Links-Rechts-Richtung, Winkelrichtung oder einer Kombination daraus relativ zum Griff (10) steuert.

5. Vorrichtung nach einem der vorstehenden Ansprüche, wobei das Steuerteil (14) Drehung des steuerbaren Kanals (38A, 38B) relativ zum Lumen (37A, 37B) steuert.

6. Vorrichtung nach einem der vorstehenden Ansprüche, wobei das Steuerteil (14) den steuerbaren Kanal (38A, 38B) so steuert, dass er sich in Proximal-Distal-Richtung relativ zum Lumen (37A, 37B) bewegt.

7. Vorrichtung nach einem der vorstehenden Ansprüche, ferner mit einem Endeffektor (52, 62) benachbart zu einem distalen Ende des steuerbaren Kanals (38A, 38B), wobei das Steuerteil (14) mit dem Endeffektor (52, 62) selektiv in Eingriff bringbar ist, um den Endeffektor (52, 62) zu aktivieren.

8. Vorrichtung nach Anspruch 7, wobei das Steuerteil (14) Bewegung des Endeffektors (52, 62) relativ zum steuerbaren Kanal (38A, 38B) steuert.

9. Vorrichtung nach einem der Ansprüche 1 bis 6, die ferner aufweist:
ein Werkzeug (50, 60) im steuerbaren Kanal (38A, 38B), wobei das Werkzeug einen Endeffektor (52, 62) an einem distalen Ende (51, 61) des Werkzeugs aufweist,
wobei das Werkzeug (50, 60) im steuerbaren Kanal (38A, 38B) in distaler Richtung beweglich ist, um den Endeffektor (52, 62) aus dem steuerbaren Kanal (38A, 38B) auszuschieben, und in proximaler Richtung, um das Werkzeug (50, 60) in den steuerbaren Kanal (38A, 38B) einzuziehen, und
wobei das Steuerteil (14) mit dem Werkzeug (50, 60) selektiv in Eingriff bringbar ist, um Bewegung des Werkzeugs (50, 60) zu steuern und den Endeffektor (52, 62) zu aktivieren.

10. Vorrichtung nach einem der vorstehenden Ansprüche, wobei der Selektor (16) mehrere Knöpfe aufweist und das Steuerteil (14) durch Aktivieren eines der mehreren Knöpfe selektiv in Eingriff gebracht wird.

11. Vorrichtung nach einem der vorstehenden Ansprüche,
wobei das Steuerteil (14) auf einer Oberseite des Griffs (10) beweglich angebracht und mit mehreren Sensoren (19) betreibbar ist, um ein Richtungssignal zu erzeugen, wenn das Steuerteil (14) bewegt wird,
wobei der Selektor (16) so konfiguriert ist, dass er ein Umschaltsignal erzeugt, um das Steuerteil (14) mit der steuerbaren Biegung (36A, 36B) oder dem steuerbaren Kanal (38A, 38B) selektiv in Eingriff zu bringen, und
wobei die Vorrichtung ferner einen Prozessor (26) aufweist, der so konfiguriert ist, dass er die steuerbare Biegung (36A, 36B) oder den steuerbaren Kanal (38A, 38B) als Reaktion auf das Richtungssignal und das Umschaltsignal aktiviert.

12. Vorrichtung nach Anspruch 11, ferner mit einer Stromquelle (28) für die mehreren Sensoren (19), den Prozessor (26), die steuerbare Biegung (36A, 36B) und den steuerbaren Kanal (38A, 38B).

13. Vorrichtung nach einem der vorstehenden Ansprüche, wobei die steuerbare Biegung (36A, 36B) einen ersten Aktor (39A, 39B) hat und der steuerbare Kanal (38A, 38B) einen zweiten Aktor (42A, 42B) hat und wobei die Vorrichtung so konfiguriert ist, dass sie den ersten Aktor (39A, 39B) oder den zweiten Aktor (42A, 42B) in Abhängigkeit von einem Umschaltsignal vom Selektor (16) selektiv aktiviert.

14. Vorrichtung nach einem der vorstehenden Ansprüche, ferner mit einer Abbildungsvorrichtung (70) an einer distalen Stirnfläche des Schafts (30), wobei das Steuerteil (14) mit der Abbildungsvorrichtung (70) selektiv in Eingriff bringbar ist, um die Abbildungsvorrichtung (70) zu aktivieren.

15. Vorrichtung nach einem der vorstehenden Ansprüche, wobei der Griff (10) zur Einhandbedienung konfiguriert ist.

## Revendications

1. Dispositif endoscopique, le dispositif comprenant:
une tige (30) s'étendant entre une extrémité distale et une extrémité proximale (32), la tige (30) incluant un coude pouvant être commandé (36A, 36B), une lumière (37A, 37B) et un canal pouvant être commandé (38A, 38B) disposé de manière à pouvoir être déplacé dans la lumière (37A, 37B);
une poignée (10) à l'extrémité proximale (32) de la tige (30), la poignée (10) incluant un organe de commande (14) pouvant être enclenché sélectivement avec le coude pouvant être commandé (36A, 36B) et le canal pouvant être commandé (38A, 38B); et
un sélecteur (16) pour enclencher sélectivement l'organe de commande (14) avec le coude pouvant être commandé (36A, 36B) ou le canal pouvant être commandé (38A, 38B).

2. Dispositif selon la revendication 1, où l'organe de commande (14) peut être enclenché sélectivement avec le coude pouvant être commandé (36A, 36B) lorsqu'il n'est pas enclenché avec le canal pouvant être commandé (38A, 38B) et peut être enclenché sélectivement avec le canal pouvant être commandé (38A, 38B) lorsqu'il n'est pas enclenché avec le coude pouvant être commandé (36A, 36B).

3. Dispositif selon l'une quelconque des revendications précédentes, où l'organe de commande (14) est un organe de commande discret, unique pouvant être actionné d'une seule main.

4. Dispositif selon l'une quelconque des revendications précédentes, où l'organe de commande (14) commande le mouvement du coude pouvant être commandé (36A, 36B) dans une direction supérieure-inférieure, une direction gauche-droite, une direction angulaire, ou une combinaison de celles-ci par rapport à la poignée (10).

5. Dispositif selon l'une quelconque des revendications précédentes, où l'organe de commande (14) commande la rotation du canal pouvant être commandé (38A, 38B) par rapport à la lumière (37A, 37B).

6. Dispositif selon l'une quelconque des revendications précédentes, où l'organe de commande (14) commande le canal pouvant être commandé (38A, 38B) pour se déplacer dans une direction proximale-distale par rapport à la lumière (37A, 37B).

7. Dispositif selon l'une quelconque des revendications précédentes, comprenant en outre un effecteur d'extrémité (52, 62) adjacent à une extrémité distale du canal pouvant être commandé (38A, 38B), où l'organe de commande (14) peut être enclenché sélectivement avec l'effecteur d'extrémité (52, 62) pour activer l'effecteur d'extrémité (52, 62).

8. Dispositif selon la revendication 7, où l'organe de commande (14) commande le mouvement de l'effecteur d'extrémité (52, 62) par rapport au canal pouvant être commandé (38A, 38B).

9. Dispositif selon l'une quelconque des revendications 1 à 6, comprenant en outre:
un outil (50, 60) dans le canal pouvant être commandé (38A, 38B), l'outil incluant un effecteur d'extrémité (52, 62) à une extrémité distale (51, 61) de l'outil,
où l'outil (50, 60) peut être déplacé à l'intérieur du canal pouvant être commandé (38A, 38B) dans une direction distale pour étendre l'effecteur d'extrémité (52, 62) hors du canal pouvant être commandé (38A, 38B) et une direction proximale pour rétracter l'outil (50, 60) dans le canal pouvant être commandé (38A, 38B), et
où l'organe de commande (14) peut être enclenché sélectivement avec l'outil (50, 60) pour commander le mouvement de l'outil (50, 60) et activer l'effecteur d'extrémité (52, 62).

10. Dispositif selon l'une quelconque des revendications précédentes, où le sélecteur (16) comprend une pluralité de boutons, et l'organe de commande (14) est enclenché sélectivement par activation de l'un de la pluralité de boutons.

11. Dispositif selon l'une quelconque des revendications précédentes,
où l'organe de commande (14) est monté de manière à pouvoir être déplacé sur une surface supérieure de la poignée (10) et peut être actionné avec une pluralité de capteurs (19) pour générer un signal directionnel lorsque l'organe de commande (14) est déplacé,
où le sélecteur (16) est configuré pour générer un signal de commutation pour enclencher sélectivement l'organe de commande (14) avec le coude pouvant être commandé (36A, 36B) ou le canal pouvant être commandé (38A, 38B), et
où le dispositif comprend en outre un processeur (26) configuré pour activer le coude pouvant être commandé (36A, 36B) ou le canal pouvant être commandé (38A, 38B) en réponse au signal directionnel et au signal de commutation.

12. Dispositif selon la revendication 11, comprenant en outre une source d'énergie (28) pour la pluralité de capteurs (19), le processeur (26), le coude pouvant être commandé (36A, 36B) et le canal pouvant être commandé (38A, 38B).

13. Dispositif selon l'une quelconque des revendications précédentes, où le coude pouvant être commandé (36A, 36B) a un premier actionneur (39A, 39B) et le canal pouvant être commandé (38A, 38B) a un second actionneur (42A, 42B), et où le dispositif est configuré pour activer sélectivement le premier actionneur (39A, 39B) ou le second actionneur (42A, 42B) en fonction d'un signal de commutation provenant du sélecteur (16).

14. Dispositif selon l'une quelconque des revendications précédentes, comprenant en outre un dispositif d'imagerie (70) sur une face distale de la tige (30), où l'organe de commande (14) peut être enclenché sélectivement avec le dispositif d'imagerie (70) pour activer le dispositif d'imagerie (70).

15. Dispositif selon l'une quelconque des revendications précédentes, où la poignée (10) est configurée pour un actionnement d'une seule main.
